# EUROPÄISCHE PATENTSCHRIFT

(11) **EP 0 656 896 B1**
(45) Veröffentlichungstag und Bekanntmachung des Hinweises auf die Patenterteilung: **17.12.1997**
(21) Anmeldenummer: 93919109.4
(22) Anmeldetag: 19.08.1993
(51) Int. Cl.: C07D 317/22, C07C 69/736, C07D 405/12, C07C 59/68, C07C 59/90, A61K 31/335

(54) **NEUE LEUKOTRIEN-B4-ANTAGONISTEN, VERFAHREN ZU IHRER HERSTELLUNG UND IHRE VERWENDUNG ALS ARZNEIMITTEL**
NEW LEUKOTRIENE-B4 ANTAGONISTS, PROCESS FOR PREPARING THE SAME AND THEIR USE AS MEDICAMENTS
NOUVEAUX ANTAGONISTES DES LEUCOTRIENES B4, LEUR PROCEDE DE PREPARATION ET LEUR UTILISATION COMME MEDICAMENTS

(30) Priorität: 25.08.1992 DE 4228201
(43) Veröffentlichungstag der Anmeldung: 14.06.1995
(73) Patentinhaber: SCHERING AKTIENGESELLSCHAFT, 13342 Berlin (DE)
(72) Erfinder: BUCHMANN, Bernd, D-10557 Berlin (DE); SKUBALLA, Werner, D-13465 Berlin (DE); HEINDL, Josef, D-14129 Berlin (DE); FRÖHLICH, Wolfgang, D-10709 Berlin (DE); EKERDT, Roland, D-13469 Berlin (DE); GIESEN, Claudia, D-13587 Berlin (DE)
(86) Internationale Anmeldenummer: EP9302225
(87) Internationale Veröffentlichungsnummer: WO9404522

(56) Entgegenhaltungen:
- EP-A- 0 104 885
- EP-A- 0 306 959
- EP-A- 0 375 457

## Beschreibung

Die Erfindung betrifft neue Leukotrien-B₄-Antagonisten, Verfahren zu ihrer Herstellung und ihre Verwendung als Arzneimittel.

Leukotrien B₄ (LTB₄) wurde 1979 von B. Samuelsson und Mitarbeiter als Metabolit der Arachidonsäure entdeckt. Bei der Biosynthese wird durch das Enzym 5-Lipoxygenase zunächst als zentrales Zwischenprodukt das Leukotrien A₄ gebildet, das dann durch eine spezifische Hydrolase in das LTB₄ umgewandelt wird.

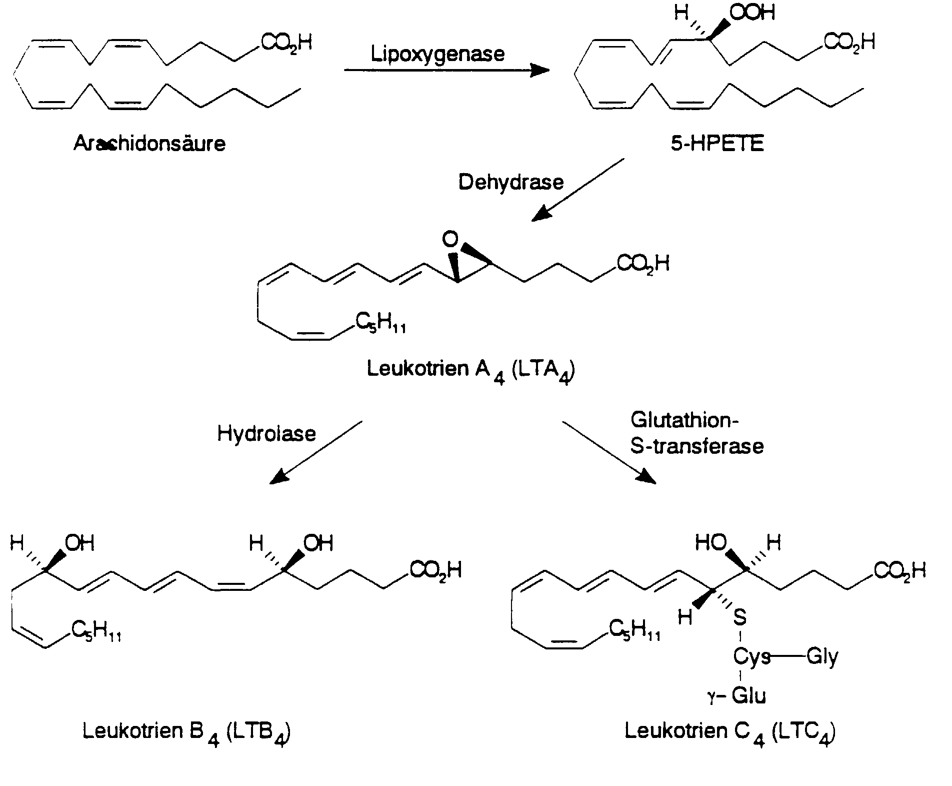

Die Nomenklatur der Leukotriene kann folgenden Arbeiten entnommen werden:
a) B. Samuelsson et. al., Prostaglandins 19 645 (1980); 17 785 (1979).
b) C.N. Serhan et al., Prostaglandins 34 201 (1987).

Die physiologische und insbesondere die pathophysiologische Bedeutung des Leukotrien B₄ wird in einigen neueren Arbeiten zusammengefaßt: a) The Leukotrienes, Chemistry and Biology eds. L.W. Chakrin, D.M. Bailey, Academic P;ess 1984. b) J.W. Gillard et al., Drugs of the Future 12 453 (1987). c) B. Samuelsson et al., Science 237, 1171 (1987). d) C.W. Parker, Drug Development Research 10 277 (1987). Hieraus ergibt sich, daß LTB₄ ein wichtiger Entzündungsmediator für entzündliche Erkrankungen ist, bei denen Leukozyten in das erkrankte Gewebe einwandern. Die Effekte von LTB₄ werden auf zellulärer Ebene durch Bindung von LTB₄ an einen spezifischen Rezeptor ausgelöst.

Von LTB₄ weiß man, daß es die Adhäsion von Leukozyten an die Blutgefäßwand verursacht. LTB₄ ist chemotaktisch wirksam, d.h. es löst eine gerichtete Wanderung von Leukozyten in Richtung eines Gradienten steigender Konzentration aus. Außerdem verändert es aufgrund seiner chemotaktischen Aktivität indirekt die vasculäre Permeabilität, wobei ein Synergismus mit Prostaglandin E₂ beobachtet wird. LTB₄ spielt offensichtlich bei entzündlichen, allergischen und immunologischen Prozessen eine entscheidende Rolle.

Leukotriene und insbesondere LTB₄ sind an Hauterkrankungen beteiligt, die mit entzündlichen Prozessen (erhöhte Gefäßpermeabilität und Ödembildung, Zellinfiltration), erhöhter Proliferation der Hautzellen und Juckreiz einhergehen, wie beispielsweise bei Ekzemen, Erythemen, Psoriasis, Pruritus und Akne. Pathologisch erhöhte Leukotrien-Konzentrationen sind an der Entstehung vieler Dermatitiden entweder ursächlich beteiligt, oder es besteht ein Zusammenhang zwischen der Persistenz der Dermatitiden und den Leukotrienen. Deutlich erhöhte Leukotrienkonzentrationen wurden beispielsweise in der Haut von Patienten mit Psoriasis oder atopischer Dermatitis gemessen.

Leukotriene und insbesondere LTB ₄ sind auch an Erkrankungen innerer Organe beteiligt, für die eine akute oder chronische entzündliche Komponente beschrieben wurde, z. B.: Gelenkerkrankungen (Athritis); Erkrankungen des Respirationstraktes (Asthma Rhinitis und Allergien); entzündliche Darmerkrankungen (Colitis); sowie Reperfusionsschäden (an Herz-, Darm- oder Nierengewebe), die durch zeitweisen krankhaften Verschluß von Blutgefäßen entstehen.

Weiterhin sind Leukotriene und insbesondere LTB₄ bei der Erkrankung an multipler Sklerose beteiligt und bei dem klinischen Erscheinungsbild des Schocks (ausgelöst durch Infektionen, Verbrennungen oder bei Komplikationen bei der Nierendialyse oder anderen extra besprochenen Perfusionstechniken).

Leukotriene und insbesondere LTB₄ haben weiterhin einen Einfluß auf die Bildung von weißen Blutkörperchen im Knochenmark, auf das Wachstum von glatten Muskelzellen, vom Keratinozyten und von B-Lymphozyten. LTB₄ ist daher bei Erkrankungen mit entzündlichen Prozessen und bei Erkrankungen mit pathologisch gesteigerter Bildung und Wachstum von Zellen beteiligt.

Erkrankungen mit diesem Erscheinungsbild stellen z. B. Leukemie oder Artherosklerose dar.

Durch die Antagonisierung der Effekte insbesondere von LTB₄ sind die Wirkstoffe und deren Darreichungsformen dieser Erfindung spezifische Heilmittel bei der Erkrankung von Mensch und Tier, bei denen insbesondere Leukotriene eine pathologische Rolle spielen.

Neben therapeutischen Möglichkeiten, die sich aus einer Antagonisierung der LTB₄- Wirkung mit LTB₄-Analoga ableiten lassen, konnte auch die Nützlichkeit und potentielle Anwendung von Leukotrien B₄-Agonisten zur Behandlung von Pilzerkrankungen der Haut gezeigt werden (H. Katayama, Prostaglandins 34 797 (1988)).

Aus EP 405116 sind bereits Verbindungen mit einer Phenylpropionsäure-Struktur, die Leukotnen-B₄ -antagonistische Eigenschaften besitzen, bekannt.

Es wurden Verbindungen gefunden, die die Wirkung des natürlichen LTB₄ überraschend stark antagonisieren.

Die Erfindung betrifft Leukotrien-B₄ -Antagonisten der Formel I, 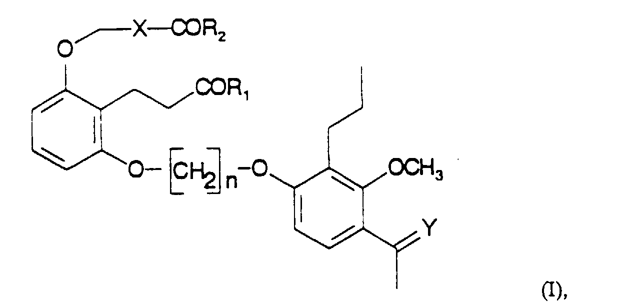 worin
- n: eine ganze Zahl von 2 bis 5,
- X: eine direkte Bindung, 1 bis 6 Methyleneinheiten, ein ortho-, meta- oder parasubstituierter Phenylring oder ein meta- oder para substituierter Pyridinring,
- Y: eine Bindung zu einem Wasserstoffatom und gleichzeitig zu einer Hydroxy gruppe, ein doppelt gebundenes Sauerstoffatom oder -O-CH₂-CH₂-O-,
- R₁ und R₂: den Rest OH, -O-(C₁-C₄)-Alkyl, O-(C₃-C₆)-Cycloalkyl, -O-(C₅-C₁₀)-Aryl, -O-(C₇-C₁₂)- Aralkyl, O-(CH₂)-CO-(C₆-C₁₀)-Aryl oder den Rest NHR₃ mit R₃ in der Bedeutung Wasserstoff, (C₁-C₄)-Alkyl, (C₃-C₆)-Cycloalkyl oder (C₇-C₁₂)- Aralkyl darstellen sowie deren Salze mit physiologisch verträglichen Basen und deren Cyclodextrinclathrate.

R₁ und R₂ als C₁-C₄-Alkoxy-Gruppe können bedeuten: Methoxy, Ethoxy, n-Propoxy, Isopropoxy, n-Butoxy, sek.-Butoxy und tert.-Butoxy.

Als Reste (C₃-C₆)-Cycloalkyl in der Definition von R₁ und R₂ kommen Cyclopropyl, Cyclobutyl, Cyclopentyl, Cyclohexyl in Betracht.

Als Reste (C₆-C₁₀)-Aryl in der Definition von R₁ und R₂ kommen Phenyl, 1-Naphthyl, 2-Naphthyl in Betracht.

Als Reste O-(CH₂)-CO-(C₆-C₁₀)-Aryl in der Definition von R₁ und R₂ kommt beispielsweise Phenacyl in Betracht.

Die Reste (C₇-C₁₂₎-Aralkyl in den Definitionen von R₁ und R₂ schließlich stellen die folgenden Gruppen dar: Benzyl, Phenethyl, 3-Phenylpropyl, 4-Phenylbutyl, 1-Methyl-3-phenylpropyl, 1-Methyl-2-phenyl-ethyl usw.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin usw.

Um zu den Cyclodextrinclathraten zu gelangen, werden die Verbindungen der Formel I mit α-, β-, oder γ-Cyclodextrin umgesetzt. Bevorzugt sind die β-Cydodextrindathrate. Die Erfindung betrifft außerdem ein Verfahren zur Herstellung von Leukotrien-B₄-Antagonisten der Formel I, dadurch gekennzeichnet, daß man in an sich bekannter Weise eine Verbindung der Formel II, 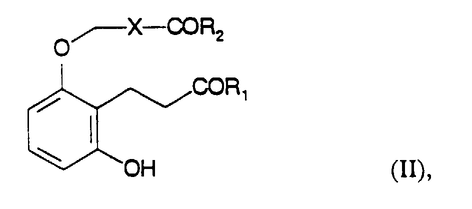 worin X, R₁ und R₂ die oben angegebene Bedeutung haben, mit einer Verbindung der Formel III, 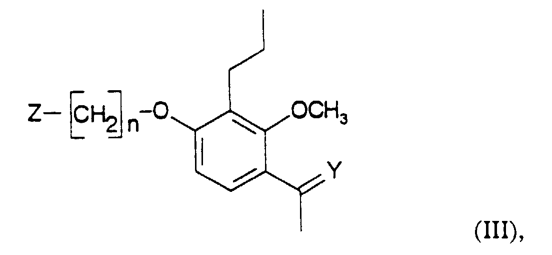 worin n und Y die oben angegebene Bedeutung hat, und Z entweder ein Halogenatom oder eine Sulfonat, wie beispielsweise Tosylat, Mesylat oder Trifluorsulfonat sein kann, ist, in Gegenwart von Cäsium, Lithium- oder Kaliumcarbonat umsetzt und gegebenenfalls die Schutzgruppen spaltet, das Methylketon reduziert, die Estergruppen verseift, Carboxylgruppen verestert oder die erhaltenen Säuren der Formel I mit organischen oder anorganischen Basen oder Cyclodextrinen umsetzt.

Das oben genannte Verfahren (II + III => I) wird in organischen Lösungsmitteln, wie z.B. Dimethylformamid, bei Temperaturen zwischen 0°C und 100°C, vorzugsweise bei Temperaturen zwischen 20°C und 60°C unter Rühren im Verlaufe von 5-24 Stunden in Gegenwart von Cäsium-, Lithium- oder Kaliumcarbonat durchgeführt.

Die Reduktion der Carbonylgruppe (Y = O) erfolgt vorzugsweise mit Natriumborhydrid unter den üblichen Bedingungen. Die erhaltenen Hydroxyverbindungen können gewünschtenfalls in die optischen Antipoden aufgetrennt werden.

Die Verseifung der Ester der Formel I wird nach den dem Fachmann bekannten Methoden durchgeführt, wie beispielsweise mit basischen Katalysatoren.

Die Einführung der Estergruppe -COR₁, bei welcher R₁ eine O-Alkylgruppe mit 1-4 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterungen mit Diazokohlenwasserstoffen erfolgt z. B. dadurch, daß man eine Lösung des Diazokohlenwasserstoffes in einem inerten Lösungsmittel, vorzugsweise in Diethylether, mit der Carboxyverbindung in dem gleichen oder in einem anderen Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seite 389 - 394 (1954)].

Die Einführung der Estergruppe -COR₁, bei welcher R₁ eine O-Arylgruppe darstellt, erfolgt nach den dem Fachmann bekannten Methoden. Beispielsweise werden die Carboxyverbindungen mit den entsprechenden Arylhydroxyverbindungen mit Dicyclohexylcarbodiimid in Gegenwart einer geeigneten Base, beispielsweise Pyridin, DMAP, Triethylamin, Diazabicyclononan (DBN), Diazabicycloundecan (DBU), in einem inerten Lösungsmittel umgesetzt. Als Lösungsmittel kommen Methylenchlorid, Ethylenchlorid, Chloroform, Essigester, Tetrahydrofuran, vorzugsweise Chloroform in Frage. Die Reaktion wird bei Temperaturen zwischen -30°C und +50°C, vorzugsweise bei 10°C, durchgeführt.

Die Leukotrien-B₄-Antagonisten der Formel I mit R₁ in der Bedeutung einer COOH-Gruppe können mit geeigneten Mengen der entsprechenden anorganischen Basen unter Neutralisierung in ein Salz überführt werden. Beispielsweise erhält man beim Lösen der entsprechenden Säuren in Wasser, das die stöchiometrische Menge der Base enthält, nach Abdampfen des Wassers oder nach Zugabe eines mit Wasser mischbaren Lösungsmittels, z.B. Alkohol oder Aceton, das feste anorganische Salz.

Zur Herstellung eines Aminsalzes wird die LTB₄-Säure z.B. in einem geeigneten Lösungsmittel, beispielsweise Ethanol, Aceton, Diethylether, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Einführung der Amidgruppe CONHR₃ erfolgt nach den dem Fachmann bekannten Methoden. Die Carbonsäuren der Formel I (R₁ = OH) werden zunächst in Gegenwart eines tertiären Amins, wie beispielsweise Triethylamin, mit Chlorameisensäureisobutylester in das gemischte Anhydrid überführt. Die Umsetzung des gemischten Anhydrids mit dem Alkalisalz des entsprechenden Amins oder mit Ammoniak erfolgt in einem inerten Lösungsmittel oder Lösungsmittelgemisch, wie beispielsweise Tetrahydrofuran, Dimethoxyethan, Dimethylformamid, Hexamethylphosphorsäuretriamid, bei Temperaturen zwischen -30°C und +60°C, vorzugsweise bei 0°C bis 30°C.

Die als Ausgangsmaterial dienenden Verbindungen der allgemeinen Formel II können beispielsweise hergestellt werden indem man in an sich bekannter Weise 2,6-Dimethoxybenzaldehyd mit (Methoxycarbonylmethylen)-triphenylphosphoran in einer Wittig-Reaktion umsetzt und durch anschließende Hydrierung mit Palladium auf Kohle in einer Wasserstoffatmosphäre in den Ester der Formel IV überführt.

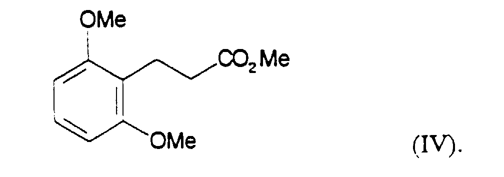

Durch Reaktion aller Methylgruppen in IV mit 48%iger HBr und in situ Lactonisierung wird das Phenol der Formel V erhalten 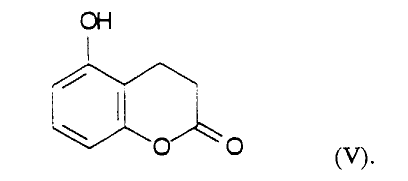 Das Phenol der Formel V wird anschließend mit einem Halogenid der Formel VI 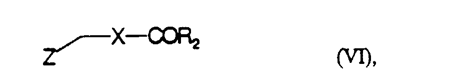 worin Z entweder Chlor, Brom oder Iod sein kann und X und R₂ die oben angegebene Bedeutung hat unter basischen Bedingungen, wie beispielsweise Casium- oder Kaliumcarbonat zu den Verbindungen der allgemeinen Formel VII umgesetzt.

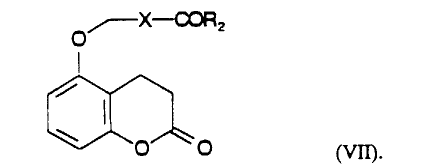

Die sich anschließende Öffnung der Lactone VII erfolgt in dem gewünschten Alkohol unter saurer Katalyse, wie beispielsweise unter Schwefelsäure, man erhält hierbei die gewünschten Verbindungen der allgemeinen Formel II.

Die Herstellung von Verbindungen der Formel III sei anhand des folgenden Syntheseweges erläutert:

### 1) 2-Hydroxy-3-propyl-4(3-Chlorpropyloxy)-acetophenon

Zu einer Lösung von 7.5 g 2,4-Dihydroxy-3-propyl-acetophenon in 45 ml DMF gibt man unter Argon 6.68 g Kaliumcarbonat gefolgt von 3.8 ml 1-Brom-3-chlorpropan und rührt anschließend 16 Stunden bei 24°C. Dann gibt man 50 ml Wasser zu und extrahiert mit dreimal je 50 ml Ether. Die vereinigten organischen Phasen wäscht man einmal mit gesättigter Natriumchlorid-Lösung, trocknet über Natriumsulfat und engt nach Filtration im Vakuum ein. Den so erhaltenen Rückstand reinigt man durch Chromatographie an Kieselgel. Mit Hexan / 0-30% Essigester erhält man 3.9 g der Titelverbindung als farbloses Öl.
IR (CHCl₃): 3005, 2965, 2935, 2873, 1627, 1583, 1498, 1470, 1418, 1371, 1268, 1120, 1057 cm⁻¹.

### 2) 2-Hydroxy-3-propyl-4(3-iodpropyloxyl)-acetophenon

Zu einer Lösung von 3.9 g der vorstehend hergestellten Chlorverbindung in 40 ml Aceton gibt man unter Argon 17.27 g Natriumiodid und rührt 10 Stunden bei 50°C. Anschließend wird das Reaktionsgemisch im Vakuum eingeengt und der Rückstand in Wasser / Ether wieder aufgenommen. Nach Phasentrennung wäscht man die organische Phase einmal mit gesättigter Natriumchlorid-Lösung, trocknet über Natriumsulfat und engt nach Filtration im Vakuum ein. Die so erhaltenen 5.05 g der gelben festen Titelverbindung werden ohne weitere Reinigung für den nächsten Reaktionsschritt verwendet.
IR (CHCl₃): 3005, 2963, 2935, 2875, 1627, 1585, 1499, 1467, 1419, 1371, 1270, 1119, 1048 cm⁻¹.

### 3) 2-Methoxy-3-propyl-4-(3-iodpropyloxy)-acetophenon

Zu einer Lösung von 3.9 g der vorstehend hergestellten Iodverbindung in 25 ml DMF gibt man unter Argon bei 24°C 2.97 g Kaliumcarbonat und 3 ml Methyliodid und rührt bei dieser Temperatur für 18 Stunden. Man verdünnt mit 100 ml Ether, wäscht einmal mit Wasser, zweimal mit gesättigter Natriumchlorid-Lösung, trocknet über Natriumsulfat und engt nach Filtration im Vakuum ein. Den so erhaltenen Rückstand reinigt man durch Chromatographie an Kieselgel. Mit Hexan / 0-20% Essigester erhält man 2.41 g der Titelverbindung als farbloses Öl.
IR (CHCl₃): 3005, 2968, 2938, 2878, 1673, 1593, 1468, 1416, 1363, 1265, 1118, 1052, 1018 cm⁻¹.

### 4) 2-Methoxy-3-propyl-4-(3-iodpropyloxyl-acetophenon-ethylenketal

Zu einer Lösung von 5.25 g des vorstehend hergestellten Acetophenonderivates in 50 ml Toluol gibt man 3.1 ml Ethylenglykol und 50 mg para-Toluolsulfonsäure. Nach Erhitzen von 6 Stunden am Wasserabscheider wird mit 80 ml Ether verdünnt, einmal mit gesättigter Natriumhydrogencarbonat-Lösung und einmal mit gesättigter Natriumchlorid-Lösung gewaschen. Nach dem Trocknen über Natriumsulfat und Filtration engt man im Vakuum ein. Den so erhaltenen Rückstand reinigt man durch Chromatographie an Kieselgel. Mit Hexan / 0-40% Ether erhält man 3.5 g der Titelverbindung als farbloses Öl.
IR (CHCl₃): 3005, 2970, 2942, 2880, 1597, 1468, 1413, 1376, 1265, 1185, 1115, 1046 cm⁻¹.

Die Verbindungen der Formel I wirken antientzündlich, antiallergetisch und antiproliferativ. Daneben besitzen sie antimykotische Eigenschaften. Folglich stellen die neuen Leukotrien-B₄-Derivate der Formel I wertvolle pharmazeutische Wirkstoffe dar. Die Verbindungen der Forml I sind besonders zur topischen Applikation geeignet, da sie eine Dissoziation zwischen erwünschter topischer Wirksamkeit und unerwünschten systemischen Nebenwirkungen aufweisen.

Die neuen Leukotden-B₄-Derivate der Formel I eignen sich in Kombination mit den in der galenischen Pharmazie üblichen Hilfs- und Trägermitteln zur lokalen Behandlung von Erkrankungen der Haut, bei denen Leukotriene eine wichtige Rolle spielen, z. B.: Kontaktdermatitis, Ekzemen der verschiedensten Art, Neurodermatosen, Erythrodermie, Pruritis vulvae et ani, Rosacea, Erythematodes cutaneus, Psoriasis, Lichen ruber planus et verrucosus und ähnlichen Hauterkrankungen.

Außerdem sind die neuen Leukotrien-B₄-Antagonisten zur Behandlung der multiplen Sklerose und der Symptome des Schocks geeignet.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Salben, Cremes oder Pflaster, überführt.

In den so formulierten Arzneimitteln ist die Wikstoffkonzentration von der Applikationsform abhängig. Bei Lotionen und Salben wird vorzugsweise eine Wirkstoffkonzentration von 0,0001% bis 3% verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Leukotrien-B₄-Derivate auch in Form von Kapseln, Tabletten oder Dragees, die vorzugsweise 0,1 bis 100 mg Wirkstoff enthalten oder oral appliziert werden oder in Form von Suspensionen, die vorzugseise 1-200 mg Wirkstoff pro Dosiseinheit enthalten und rektal appliziert werden auch zur Behandlung von Erkrankungen innerer Organe, bei denen Leukotriene eine wichtige Rolle spielen, wie z. B.: allergische Erkrankungen des Darmtraktes, wie der Kolitis ulcerosa und der Kolitis granulomatosa.

In diesen neuen Applikationformen eignen sich die neuen LTB₄-Derivate neben der Behandlung von Erkrankungen innerer Organe mit entzündlichen Prozessen auch zur Behandlung von Erkrankungen bei denen leukotrienabhängig das gesteigerte Wachstum und die Neubildung von Zellen im Vordergrund stehen. Beispiele sind Leukämie (gesteigertes Wachstum weißer Blutkörperchen) oder Artherosklerose (gesteigertes Wachstum glatter Muskelzellen von Blutgefaßen).

Die neuen Leukotrien-B₄-Derivate können auch in Kombination, wie z. B. mit Lipoxygenasehemmern, Cyclooxygenasehemmern, Glukokortikoiden, Prostacyclinagonisten, Thromboxanantagonisten, Leukotrien D₄-antagonisten, Leukotrien E₄-antagonisten, Leukotrien F₄-antagonisten, Phosphodiesterasehemmem, Calciumantagonisten, PAF-Antagonisten oder anderen bekannten Therapieformen der jeweiligen Erkrankungen, verwendet werden.

Die nachfolgenden Ausführungsbeispiele dienen zur näheren Erläuterung des erfindungsgemäßen Verfahrens.

### Beispiel 1

### 5- {2-(2-Ethoxycarbonylethyl)-3-[3-(4-(2-methyl-1,3-dioxolan-2-yl-3-methoxy-2-propylphenoxyl-propoxy]-phenoxy}-pentansäureethylester

Zu einer Lösung von 1.37 g 5-[2-(2-Ethoxycarbonylethyi)-3-hydroxy-phenoxy]-pentansäureethylester in 15 ml DMF gibt man unter Stickstoff bei 24°C 1.45 g Cäsiumcarbonat und 1.80 g 2-Methoxy-3-propyl-4-(3-iodpropyloxy)-acetophenon-ethylenketal und rührt bei dieser Temperatur 24 Stunden. Das Reaktionsgemisch wird anschließend auf 50 ml Eiswasser gegeben. Man extrahiert dreimal mit je 50 ml Essigester, wäscht die vereinigten organischen Phasen einmal mit gesättigter Natriumchlorid-Lösung, trocknet über Natriumsulfat und engt nach Filtration im Vakuum ein. Den so erhaltenen Rückstand reinigt man durch Chromatographie an Kieselgel. Mit Hexan /0-30% Essigester erhält man 1.03 g der Titelverbindung als farbloses Öl.
IR (CHCl₃): 3035, 2985, 2965, 2940, 2880, 1730, 1596, 1463, 1257, 1183, 1108, 1042 cm⁻¹.

Das Ausgangmaterial für die obige Titelverbindung wird wie folgt hergestellt:
**1a**) (2E)-3(2,6-Dimethoxyphenyl)-2-propensäuremethylester
   Zu einer Lösung von 8.48 g 2,6-Dimethoxybenzaldehyd in 450 ml Tetrahydrofuran gibt man unter Stickstoff 35.6 g (Methoxycarbonylmethylen)-triphenylphosphoran und erhitzt 16 Stunden unter Rückfluß. Man gibt auf 200 ml Eiswasser, extrahiert dreimal mit Essigester, wäscht die vereinigten organischen Phasen einmal mit gesättigter Natriumchlorid-Lösung, trocknet über Natriumsulfat und engt nach Filtration im Vakuum ein. Den so erhaltenen Rückstand reinigt man durch Chromatographie an Kieselgel. Mit Hexan / 0-40% Essigester erhält man einen Feststoff, der aus Hexan / Toluol umkristallisiert wird. Man erhält auf diese Weise 10.6 g der Titelverbindung als kristalline Substanz (Schmelzpunkt: 76-78°C).
   IR (CHCl₃): 3038, 3010, 2970, 2958, 2912, 2850, 1705, 1627, 1598, 1585, 1478. 1438, 1320, 1260, 1183, 1172, 1115 cm⁻¹.
**1b)** 3-(2,6-Dimethoxyphenyl)-propansäuremethylester
   Zu einer Lösung von 10.6 g des in Beispiel la) hergestellten Esters in 300 ml Methanol gibt man 1.12 g Palladium/Kohle (10%) und rührt in einer Wasserstoffatmosphäre für 6 Stunden. Anschließend filtriert man über Kieselgel ab und wäscht mit Essigester nach. Nach dem Einengen erhält man 10.4 g der Titelverbindung als farbloses Öl.
   IR (CHCl₃): 3038, 3003, 2957, 2910, 2841. 1730, 1598, 1475, 1450,1438,1279, 1255, 1191, 1173, 1112 cm⁻¹.
**1c**) 5-Hydroxy-3,4-dihydro-cumarin
   10.4 g des vorstehend hergestellten Esters aus Beispiel 1b) werden mit 144 ml 48%iger wäßriger HBr für 16 Stunden erhitzt (Badtemperatur 140°C). Anschließend gibt man auf 500 ml Eiswasser und sättigt die wäßrige Phase mit Natriumchlorid. Man extrahiert dreimal mit Essigester, wäscht die vereinigten organischen Phasen einmal mit gesättigter Natriumchlorid-Lösung, trocknet über Natriumsulfat und engt nach Filtration im Vakuum ein. Den so erhaltenen Rückstand reinigt man durch Umkristallisation aus Toluol / Essigester. Man erhält 4.38 g der Titelverbindung als orange gefärbte Kristalle (Schmelzpunkt 172-176°C).
   IR (CHCl₃): 3603, 3300, 3025, 2990, 2920, 2860, 1768, 1625, 1607, 1469, 1290, 1266, 1252, 1170, 1151, 1042, 1018 cm⁻¹.
**1d**) 5-(4-Ethoxycarbonylbutoxy)-3,4-dihydrocumarin
   Zu einer Lösung von 965 mg des in Beispiel 1c) hergestellten Phenols in 16 ml Dimethylformamid gibt man bei 22°C unter Sickstoff 3.83 g Cäsiumcarbonat, gefolgt von 1.87 ml 5-Brompentansäureethylester. Nach 8 Stunden rühren bei 22°C gibt man die Reaktionsmischung auf 50 ml Eiswasser.Man extrahiert dreimal mit je 40 ml Essigester, wäscht die vereinigten organischen Phasen einmal mit gesättigter Natriumchlorid-Lösung, trocknet über Natriumsulfat und engt nach Filtration im Vakuum ein. Den so erhaltenen Rückstand reinigt man durch Chromatographie an Kieselgel. Mit Hexan / 0-30% Essigester erhält man 1.42 g der Titelverbindung als farbloses Öl.
   IR (CHCl₃): 3035, 2990, 2945, 2920, 2878, 1767, 1729, 1613, 1597, 1463, 1350, 1273, 1255, 1163, 1149, 1087 cm⁻¹.
**1e)** 5-[2-(2-Ethoxycarbonylethyl)-3-hydroxy-phenoxy]-pentansäureethylester
   Zu einer Lösung von 1.42 g des in Beispiel 1d) hergestellten Esters in 20 ml Ethanol gibt man unter Stickstoff 0.45 ml konzentrierte Schwefelsäure, erwärmt zunächst 4 Stunden auf 65°C und läßt anschließend 16 Stunden bei 22°C rühren. Man gibt das Reaktionsgemisch auf 50 ml Eiswasser, extrahiert viermal mit je 30 ml Essigester, wäscht die vereinigten organischen Phasen einmal mit gesättigter Natriumhydrogencarbonat-Lösung, und einmal mit einer halbgesättigten Natriumchlorid-Lösung, trocknet über Natriumsulfat und engt nach Filtration im Vakuum ein. Man erhält auf diese Weise 1.37 g der Titelverbindung als farbloses Öl, welches ohne weitere Reinigung in den nächsten Reaktionsschritt eingesetzt wird.
   iR (CHCl₃): 3330, 3035, 2985, 2960, 2943, 2878, 1728, 1712, 1610, 1594, 1470, 1378, 1277, 1185, 1161, 1092 cm⁻¹.

### Beispiel 2

### 3-{2-(3-Methoxycarbonylphenylmethyloxyl-6-[3-(4-(2-methyl-1,3-dioxolan-2-yl)-3-methoxy-2-propylphenoxyl)-propoxy]-phenyl}-propansäuremethylester

Zu einer Lösung von 3.31 g 3-[2-(3-Methoxycarbonylphenylmethyloxy)-6-hydroxyphenyl]-propansäuremethylester in 35 ml DMF gibt man unter Stickstoff bei 24°C 3.44 g Cäsiumcarbonat und 4.44 g 2-Methoxy-3-propyl-4-(3-iodpropyloxy)-acetophenon-ethylenketal und rührt bei dieser Temperatur 24 Stunden. Das Reaktionsgemisch wird anschließend auf 120 ml Eiswasser gegeben. Man extrahiert dreimal mit je 120 ml Essigester, wäscht die vereinigten organischen Phasen einmal mit gesättigter Natriumchlorid-Lösung, trocknet über Natriumsulfat und engt nach Filtration im Vakuum ein. Den so erhaltenen Rückstand reinigt man durch Chromatographie an Kieselgel. Mit Hexan / 0-40% Essigester erhält man 4.6 g der Titelverbindung als farbloses Öl.
IR (CHCl₃): 3035, 3000, 2957, 2878, 1720, 1593, 1462, 1435, 1290, 1255, 1182, 1106, 1041 cm⁻¹.

Das Ausgangmaterial für die obige Titelverbindung wird wie folgt hergestellt:
**2a)** 5-(3-Methoxycarbonylphenylmethyloxy)-3,4-dihydrocumarin
   Zu einer Lösung von 2.42 g des in Beispiel 1c) hergestellten Phenols in 40 ml Dimethylformamid gibt man bei 22°C unter Stickstoff 9.52 g Cäsiumcarbonat, gefolgt von 6.68 g 3-Brommethylbenzoesäuremethylester. Nach 17 Stunden rühren bei 22°C gibt man die Reaktionsmischung auf 200 ml Eiswasser. Man extrahiert viermal mit je 150 ml Essigester, wäscht die vereinigten organischen Phasen einmal mit halbgesättigter Natriumchlorid-Lösung, trocknet über Natriumsulfat und engt nach Filtration im Vakuum ein. Den so erhaltenen Rückstand reinigt man durch Chromatographie an Kieselgel. Mit Hexan /0-33% Essigester erhält man 3.05 g der Titelverbindung als farbloses Öl.
   IR (CHCl₃): 3020, 2952, 2910, 1768, 1720, 1613, 1596, 1463, 1291, 1266, 1253, 1169, 1146, 1109, 1077 cm⁻¹.
**2b)** 3-[2-3-Methoxycarbonylphenylmethyloxy)-6-hydroxyphenyl]-propansäuremethylester
   Zu einer Lösung von 3.0 g des in Beispiel 2a) hergestellten Esters in 31 ml Methanol gibt man unter Stickstoff 0.62 ml konzentrierte Schwefelsäure, erwärmt zunächst 6 Stunden auf 65°C und läßt anschließend 16 Stunden bei 22°C rühren. Man gibt man das Reaktionsgemisch auf 200 ml Eiswasser, extrahiert viermal mit je 150 ml Essigester, wäscht die vereinigten organischen Phasen einmal mit gesättigter Natriumhydrogencarbonat-Lösung, und dreimal mit einer halbgesättigten Natriumchlorid-Lösung, trocknet über Natriumsulfat und engt nach Filtration im Vakuum ein. Man erhält auf diese Weise 3.6 g der Titelverbindung als farbloses Öl, welches ohne weitere Reinigung in den nächsten Reaktionsschritt eingesetzt wird.
   IR (CHCl₃): 3605, 3340, 3035, 3010, 2968, 2880, 1718, 1612, 1592, 1470, 1440, 1370, 1293 1273, 1181, 1082 cm⁻¹.

### Beispiel 3

### 3- {2-(4-Methoxycarbonylphenylmethyloxy)-6- [3-(4(2-methyl- 1,3-dioxolan-2-yl)-3-methoxy-2-propylphenoxy)-propoxy]-phenyl} -propansäureethylester

In Analogie zu Beispiel 2) erhält man aus 440 mg des in Beispiel 3a) hergestellten Phenols und 590 mg 2-Methoxy-3-propyl-4-(3-iodpropyloxy)-acetophenon-ethylenketal 583 mg der Titelverbindung als farbloses Öl.
IR (CHCl₃): 3035, 3005, 2957, 2878, 1719, 1593, 1462, 1434, 1290, 1256, 1180, 1105, 1040 cm⁻¹.

Das Ausgangmaterial für die obige Titelverbindung wird wie folgt hergestellt:
**3a)** 5-(4-Methoxycarbonylphenylmethyloxy)-3,4-dihydrocumarin
   In Analogie zu Beispiel 2a) erhält man aus 605 mg des in Beispiel 1c) hergestellten Phenols und 1.67 g 4-Brommethylbenzoesäuremethylester 421 mg der Titelverbindung als farbloses Öl.
   IR (CHCl₃): 3012, 2952, 2910, 1767, 1720, 1612, 1597, 1465, 1437, 1282, 1252, 1170, 1147, 1110, 1072 cm⁻¹.
**3b)** 3-[2-(4-Methoxycarbonylphenylmethyloxy)-hydroxyphenyl ]-propansäure- methylester
   In Analogie zu Beispiel 2b) erhält man aus 410 mg des in Beispiel 3a) hergestellten Esters 442 mg der Titelverbindung als farbloses Öl.
   IR (CHCl₃): 3608, 3340, 3035, 3010, 2958, 2877, 1719, 1612, 1595, 1470, 1440, 1416, 1370, 1313, 1283, 1180, 1162, 1112, 1088 cm⁻¹.

### Beispiel 4

### 5-{2-(2-Ethoxycarbonylethyl)-3-[3-(4-acetyl-3-methoxy-2-propylphenoxyl-propoxy]-phenoxy}-pentansäureethylester

Zu 1.0 g der in Beispiel 1) hergestellten Titelverbindung gibt man 30 ml eines Gemisches von Essigsäure / Wasser / Tetrahydrofuran im Verhältnis 65:35:10 und rührt für 20 Stunden bei 22°C. Anschließend engt man unter Toluolzusatz im Vakuum ein. Den so erhaltenen Rückstand reinigt man durch Chromatographie an Kieselgel. Mit Hexan / 0-40% Essigester erhält man 0.46 g der Titelverbindung als farbloses Öl.
IR (CHCl₃): 3035, 3005, 2965, 2940, 2880, 1728, 1672, 1590, 1462, 1270, 1183, 1164, 1108 cm⁻¹.

### Beispiel 5

### 3-{2-(3-Methoxycarbonylphenylmethyloxyl-6-[3-(4-acetyl-3-methoxy-2-propylphenoxyl-propoxy]-phenyl }-propansäuremethylester

In Analogie zu Beispiel 4) erhält man aus 4,3 g der in Beispiel 2) hergestellten Titelverbindung 3.22 g der Titelverbindung als farbloses Öl.
IR (CHCl₃): 3035, 3005, 2958, 2875, 1721, 1670, 1590, 1462, 1435, 1290, 1267, 1183. 1165, 1110 cm⁻¹.

### Beispiel 6

### 5-{2-(2-Carboxyethyl)-3-[3-(4-acetyl-3-methoxy-2-propylphenoxy)-propoxy]-phenoxy}-pentansäure

Zu einer Lösung von 280 mg des in Beispiel 4) hergestellten Diesters in einer Mischung aus 2.5 ml Methanol und 4.5 ml Tetrahydrofuran gibt man 5 ml einer 0.5 N Lithiumhydroxid-Lösung und 120 mg festes Lithiumhydroxid. Man rührt 20 Stunden bei 22°C, gibt anschließend 10 ml Wasser hinzu und stellt mit einer 1N Schwefelsäure einen pH-Wert von 4.5 ein. Man extrahiert dreimal mit je 70 ml Essigester, wäscht die vereinigten organischen Phasen einmal mit gesättigter Natriumchlorid-Lösung, trocknet über Natriumsulfat und engt nach Filtration im Vakuum ein. Den so erhaltenen Rückstand reinigt man durch Chromatographie an Kieselgel. Mit Methylenchlorid / 0-25% Isopropanol erhält man 160 mg der Titelverbindung als farbloses Öl.
IR (CHCl₃): 3520, 3095, 3035, 3005, 2960, 2933, 2870, 1710, 1668, 1588, 1460, 1412, 1257, 1182, 1106 cm⁻¹.

### Beispiel 7

### 3-{2-(3-Carboxyphenylmethyloxy-6-[3-(4-acetyl-3-methoxy-2-propylphenoxy)- propoxy]-phenyl }-propansäure

In Analogie zu Beispiel 6) erhält man aus 339 mg der in Beispiel 5) hergestellten Titelverbindung 264 mg der Titelverbindung nach Umkristallisation aus Methylenchlorid als kristalline Verbindung (Schmelzpunkt: 125°C).
IR (CHCl₃): 3410, 3100, 3035, 3005, 2963, 2935, 2873, 1700, 1672, 1590, 1462, 1412, 1289, 1173, 1111 cm⁻¹.

### Beispiel 8

### 3- {2-(3-Carboxyphenylmethyloxy)-6-[3-(4-(2-methyl- 1,3-dioxolan-2-yl)-3-methoxy-2-propylphenoxyl-propoxy]-phenyl )-propansäure

Zu einer Lösung von 300 mg des in Beispiel 2) hergestellten Diesters in einer Mischung aus 2.3 ml Methanol und 4.1 ml Tetrahydrofuran gibt man 4.6 ml einer 0.5 N Lithiumhydroxid-Lösung und 111 mg festes Lithiumhydroxid. Man rührt 20 Stunden bei 22°C, gibt anschließend 10 ml Wasser hinzu und stellt mit einer 1N Schwefelsäure einen pH-Wert von 4.5 ein. Man extrahiert dreimal mit je 70 ml Essigester, wäscht die vereinigten organischen Phasen einmal mit gesättigter Natriumchlorid-Lösung, trocknet über Natriumsulfat und engt nach Filtration im Vakuum ein. Den so erhaltenen Rückstand reinigt man durch Chromatographie an Kieselgel. Mit Hexan / 0-40% Essigester erhält man 24 mg der Titelverbindung als farbloses Öl.
IR (KBr): 3410, 3090, 2960, 2935, 2890, 2875, 1717, 1700, 1595, 1465, 1410, 1386, 1376, 1262, 1255, 1218, 1188, 1112, 1044 cm⁻¹.

### Beispiel 9

### 3- {2-(6-Methoxycarbonylpyridin-2-yl-methyloxy)-6-[3-(4-(2-methyl-1,3-dioxolan-2-yl)-3-methoxy-2-propylphenoxy)-propoxy]-phenyl}-propansäuremethylester

Zu einer Lösung von 386 mg 3-[2-(6-Methoxycarbonylpyridin-2-yl-methyloxy)-6-hydroxyphenyl]-propansäuremethylester in 4.1 ml DMF gibt man unter Stickstoff bei 24°C 0.40 g Cäsiumcarbonat und 0.53 g 2-Methoxy-3-propyl-4-(3-iodpropyloxy)-acetophenon-ethylenketal und rührt bei dieser Temperatur 4.5 Stunden. Das Reaktionsgemisch wird anschließend auf 14 ml Eiswasser gegeben. Man extrahiert viermal mit je 100 ml Essigester, wäscht die vereinigten organischen Phasen einmal mit gesättigter Natriumchlorid-Lösung, trocknet über Natriumsulfat und engt nach Filtration im Vakuum ein. Den so erhaltenen Rückstand reinigt man durch Chromatographie an Kieselgel. Mit Hexan / 0-40% Essigester erhält man 615 mg der Titelverbindung als farbloses Öl.
IR (CHCl₃): 3036, 3005, 2955, 1728, 1588, 1410, 1257, 1110, 1057 cm⁻¹.

Das Ausgangmaterial für die obige Titelverbindung wird wie folgt hergestellt:
9a) 6-(Diphenyl-tert.-butylsilyloxymethyl)-2-hydroxymethylpyridin
   Zu einer Suspension von 7.83 g Natriumhydrid (55%ig in Mineralöl) in 350 ml Tetrahydrofuran tropft man bei 0°C unter Stickstoff 25 g 2,6-Bis-(hydroxymethyl)-pyridin gelöst in 125 ml eines Gemisches aus Tetrahydrofuran und Dimethylformamid im Verhältnis 1:1. Nach 45 min. Rühren tropft man 46.8 ml tert.-Butyldiphenylsilylchlorid zu und rührt für 2 Stunden bei 24°C. Das Reaktionsgemisch wird mit 1 1 Ether verdünnt, zweimal mit halbkonzentrierter Natriumchlorid-Lösung gewaschen, über Natriumsulfat getrocknet und nach Filtration im Vakuum eingeengt. Den so erhaltenen Rückstand reinigt man durch Chromatographie an Kieselgel. Mit Hexan /20-60% Essigester erhält man 42.8 g der Titelverbindung als farbloses Öl.
   IR (Film): 3412, 3071, 2931, 2857, 1739, 1596, 1428, 1242, 1113, 998, 824, 740 cm⁻¹.
9b) 6(Diphenyl-tert.-butylsilyloxymethyl)-2-pyridincarbaldehyd
   Zu einer Lösung von 20 g 6-(Diphenyl-tert.-butylsilyloxymethyl)-2-hydroxymethylpyridin in 236 ml Toluol gibt man bei 24°C unter Stickstoff portionsweise 100 g Braunstein und rührt 24 Stunden. Man filtriert über Celite ab, wäscht gut mit Toluol nach und engt im Vakuum ein. Man erhält 19.0 g der Titelverbindung als gefärbte Flüssigkeit, die ohne weitere Reinigung weiter eingesetzt wird.
   IR (Film): 3070, 2931, 2857, 1715, 1593, 1428, 1210, 1113, 998, 824, 741 cm⁻¹.
9c) 6-(Diphenyl-tert.-butylsilyloxymethyl)-2-pyridincarbonsäuremethylester
   Zu einer Lösung von 18.29 g 6-(Diphenyl-tert.-butylsilyloxymethyl)-2-pyridincarbaldehyd und 7.22 g Kaliumhydroxid in 593 ml Methanol tropft man bei 0°C unter Stickstoff eine Lösung von 8.15 g Iod in 148 ml Methanol zu. Nach 1 Stunde Rühren wurde nochmals Iod-Lösung zugegeben und nachgerührt und dies auch wiederholt bis in der Dünnschicht eine vollständige Reaktion erkennbar war. Das Reaktionsgemisch wird bis auf ca. 60 ml im Vakuum eingeengt, mit 100 ml halbkonzentrierter Natriumchlorid-Lösung versetzt und viermal mit Ether extrahiert. Die vereinigten organischen Phasen werden einmal mit 60 ml gesättigter Natriumthiosulfat-Lösung, halbkonzentrierter Natriumchlorid-Lösung gewaschen, über Natriumsulfat getrocknet und nach Filtration im Vakuum eingeengt. Man erhält 19.4 g der Titelverbindung, die ohne weitere Reinigung weiter eingesetzt wird.
   IR (CHCl₃): 3070, 2998, 2957, 2857, 1727, 1593, 1426, 1314, 1110, 980, 822, 700 cm⁻¹.
9d) 6-(Hydroxymethyl)-2-pyridincarbonsäuremethylester
   Eine Lösung von 3.52 g 6-(Diphenyl-tert.-butylsilyloxymethyl)-2-pyridincarbonsäuremethylester in 87 ml Tetrahydrofuran versetzt man bei 24°C unter Stickstoff mit 13.6 g Tetrabutylammoniumfluorid-trihydrat und rührt für 2 Stunden. Anschließend versetzt man mit 32 ml konzentrierter Natriumchlorid-Lösung und extrahiert viermal mit Essigester. Die vereinigten organischen Phasen werden über Natriumsulfat getrocknet und nach Filtration im Vakuum eingeengt. Den so erhaltenen Rückstand reinigt man durch Chromatographie an Kieselgel. Mit Hexan / 50-100% Essigester erhält man 0.92 g der Titelverbindung als farbloses Öl.
   IR (CHCl₃) : 3663, 3072, 3008, 2932, 2859, 1727, 1589, 1428, 1114, 821, 703 cm⁻¹.
9e) 6-(Chlormethyl)-2-pyridincarbonsäuremethylester
   Zu 2.50 g 6-(Hydroxymethyl)-2-pyridincarbonsäuremethylester gibt man langsam bei 0°C unter Stickstoff 5 ml Thionylchlorid und rührt für 1 Stunde. Das Reaktionsgemisch wird im Vakuum eingeengt. Den so erhaltenen Rückstand versetzt man mit 15 ml gesättigter Natriumhydrogencarbonat-Lösung und extrahiert dreimal mit je 25 ml Toluol. Nach dem Trocknen über Natriumsulfat und Filtration wird im Vakuum eingeengt. Man erhält 2.37 g der Titelverbindung , die ohne weitere Reinigung weiter eingesetzt wird.
   IR (CHCl₃): 3033, 3008, 2954, 1727, 1591, 1439, 1322, 1264, 1133, 1086, 994, 701 cm⁻¹.
9f) 5-(6-Methoxycarbonylpyridin-2-yl-methyloxy)-3,4-dihydrocumarin
   In Analogie zu Beispiel 2a) erhält man aus 2.10 g des in Beispiel 1c) hergestellten Phenols und 2.37 g 6-(Chlormethyl)-2-pyridincarbonsäuremethylester 3.00 g der Titelverbindung.
   Schmelzpunkt: 198°C
   IR: 3072, 3001, 2953, 1742, 1725, 1591, 1438, 1321, 1261, 1228, 1197, 1169, 1133, 1085, 994, 888, 835, 799, 747 cm⁻¹.
9g) 3-[2-(6-Methoxycarbonylpyrindin-2-yl-methyloxy)-6-hydroxyphenyl]-propansäuremethylester
   In Analogie zu Beispiel 2b) erhält man aus 350 mg des in Beispiel 9f) hergestellten Esters 390 mg der Titelverbindung als braun gefärbtes Öl.
   IR (CHCl₃): 3331, 3033, 3007, 2955, 1727, 1592, 1470, 1441, 1369, 1317, 1237. 1180, 1161, 1139, 1103, 1046, 996, 827 cm⁻¹.

### Beispiel 10

### 3-{2-6-Methoxycarbonylpyridin-2-yl-methyloxy)-6-[3-4-acetyl-3-methoxy-2-propylphenoxy)-propoxy]-phenyl }-propansäuremethylester

In Analogie zu Beispiel 4) erhält man aus 615 mg der in Beispiel 9) hergestellten Titelverbindung 479 mg der Titelverbindung als farbloses Öl.
IR (CHCl₃): 3037, 3008, 2956, 1728, 1670, 1590, 1464, 1412, 1360, 1269, 1184, 1113, 1062, 811 cm⁻¹.

### Beispiel 11

### 3-{2-(6-Carboxypyridin-2-yl-methyloxy)-6-[3-(4-acetyl-3-methoxy-2-propylphenoxy)-propoxy]-phenyl }-propansäure

In Analogie zu Beispiel 6) erhält man aus 159 mg der in Beispiel 10) hergestellten Titelverbindung 159 mg der Titelverbindung ohne Reinigung als schwach gelbliches Öl.
IR (Film): 3450, 3180, 3097, 2963, 2937, 2873, 1735, 1710, 1672, 1590, 1465, 1411. 1360, 1269, 1218, 1184, 1113, 1062, 999, 812 cm⁻¹.

### Beispiel 12

### (E/Z)-3-{2-(3-Methoxycarbonylphenylmethyloxy)-6-[3-(4-(1-methoxypropen-2-yl)-3-methoxy-2-propylphenoxy)-propoxy]-phenyl )-propansäuremethylester

Zu einer Lösung von 0.46 g Methoxymethyltriphenylphosphoniumchlorid in 2 ml eines Gemisches aus Dimethylsulfoxid und Tetrahydrofuran im Verhältnis 2:1 gibt man bei 0°C unter Stickstoff 0.14 g Kalium-tert.-butylat und rührt für 30 Minuten.
Anschließend gibt man 200 mg der in Beispiel 5) hergestellten Titelverbindung in 3.7 ml Tetrahydrofuran zu und rührt für 16 Stunden bei 24°C. Das Reaktionsgemisch wird mit halbkonzentrierter Natriumchlorid-Lösung verdünnt und dreimal mit Ether extrahiert. Nach dem Trocknen über Natriumsulfat und Filtration wird im Vakuum eingeengt. Den so erhaltenen Rückstand reinigt man durch Chromatographie an Kieselgel. Mit Hexan / 0-40% Ether erhält man 103 mg der Titelverbindung als farbloses Öl.
IR (Film): 2954, 2871, 1727, 1655, 1595, 1464, 1410, 1374, 1285, 1219, 1109, 982 cm⁻ ¹.

### Beispiel 13

### 3-{2-(3-Methoxycarbonylphenylmethyloxy)-6-[3-(4-(propen-2-yl)-3-methoxy-2-propylphenoxyl-propoxy]-phenyl }-propansäuremethylester

In Analogie zu Beispiel 12) erhält man aus 200 mg der in Beispiel 5) hergestellten Titelverbindung und 300 mg Methyltriphenylphosphoniumbromid 110 mg der Titelverbindung als farbloses Öl.
IR (Film): 2954, 2871, 1727, 1595, 1464, 1372, 1288, 1203, 1113, 975, 898 cm⁻¹.

### Beispiel 14

### (E/Z)-3-{2-(3-Carboxyphenylmethyloxyl-6-[3-(4-(1-methoxypropen-2-yl)-3-methoxy-2-propylphenoxy)-propoxyl-phenyl }-propansãure

In Analogie zu Beispiel 6) erhält man aus 97 mg der in Beispiel 12) hergestellten Titelverbindung 83 mg der Titelverbindung durch Säulenchromatographie an Kieselgel mit Hexan / 0-100% Essigester als farbloses Öl.
IR (CHCl₃): 3508, 3077, 3037, 2950, 2930, 1702, 1594, 1464, 1411, 1256, 1183, 1110 cm⁻¹.

### Beispiel 15

### 3-{2-(3-Carboxyphenylmethyloxyl-6-[3-(4-(propen-2-yl)-3-methoxy-2-propylphenoxy)-propoxy]-phenyl }-propansäure

In Analogie zu Beispiel 6) erhält man aus 100 mg der in Beispiel 13) hergestellten Titelverbindung 23 mg der Titelverbindung durch Säulenchromatographie an Kieselgel mit Hexan / 0-10 % Essigester als feste Substanz. Schmelzpunkt: 86-88°C.
IR (KBr): 3440, 3078, 2950, 2938, 1702, 1595, 1472, 1412, 1258, 1123, 895 cm⁻¹.

## Patentansprüche

1. Die Erfindung betrifft Leukotrien-B₄ -Antagonisten der Formel I, 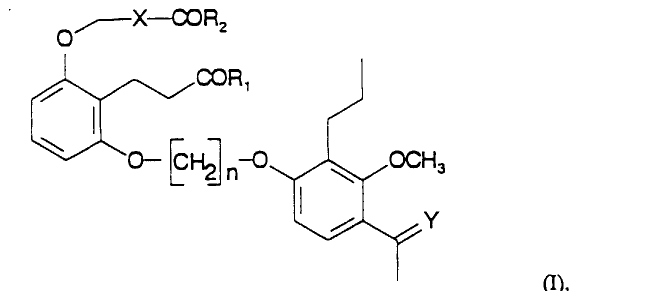 worin
n eine ganze Zahl von 2 bis 5,
X eine direkte Bindung, 1 bis 6 Methyleneinheiten, ein ortho-, meta- oder parasubstituierter Phenylring oder ein meta- oder para substituierter Pyridinring,
Y eine Bindung zu einem Wasserstoffatom und gleichzeitig zu einer Hydroxy gruppe, ein doppelt gebundenes Sauerstoffatom oder -O-CH₂-CH₂-O-,
R₁ und R₂ den Rest OH, -O-(C₁-C₄)-Alkyl, O-(C₃-C₆)-Cycloalkyl, -O-(C₆-C₁₀)-Aryl, -O-(C₇-C₁₂-Aralkyl, O-CH₂)-CO-(C₆-C₁₀)-Aryl oder den Rest NHR₃ mit R₃ in der Bedeutung Wasserstoff, (C₁-C₄)-Alkyl, (C₃-C₆)-Cycloalkyl oder (C₇-C₁₂)- Aralkyl darstellen sowie deren Salze mit physiologisch verträglichen Basen und deren Cyclodextrinclathrate.

2. Pharmazeutische Präparate gekennzeichnet durch den Gehalt an Leukotrien-B₄-Antagonisten der allgemeinen Formel I gemäß Patentanspruch 1.

3. Verfahren zur Herstellung von Leukotrien-B₄-Antagonisten der Formel I, dadurch gekennzeichnet, daß man in an sich bekannter Weise eine Verbindung der Formel II, 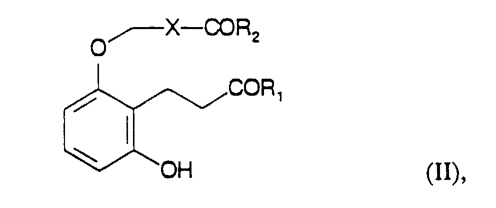 worin X, R₁ und R₂ die oben angegebene Bedeutung haben, mit einer Verbindung der Formel III, 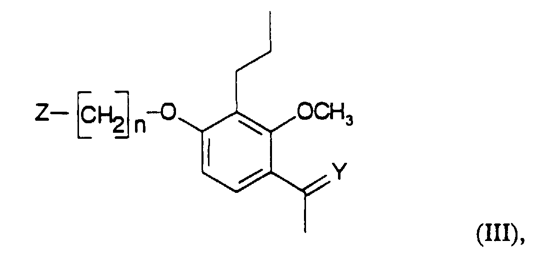 worin n und Y die oben angegebene Bedeutung hat, und Z entweder ein Halogenatom oder ein Sulfonat, wie beispielsweise Tosylat, Mesylat oder Trifluorsulfonat sein kann, ist, in Gegenwart von Cäsium, Lithium- oder Kaliumcarbonat umsetzt und gegebenenfalls die Schutzgruppen spaltet, das Methylketon reduziert, die Estergruppen verseift, Carboxylgruppen verestert oder die erhaltenen Säuren der Formel I mit organischen oder anorganischen Basen oder Cyclodextrinen umsetzt.

4. (E/Z)-3-{2-(3-Methoxycarbonylphenylmethyloxy)-6-[3-(4-(1-methoxypropen-2-yl)-3-methoxy-2-propylphenoxy)-propoxy]-phenyl}-propansäuremethylester

5. 3-{2-(3-Methoxycarbonylphenylmethyloxy)-6-[3-(4-(propen-2-yl)-3-methoxy-2-propylphenoxy)-propoxy]-phenyl}-propansäuremethylester

6. (E/Z)-3- {2-(3-Carboxyphenylmethyloxy)-6-[3-(4-(1-methoxypropen-2-yl)-3-methoxy-2-propylphenoxy)-propoxy]-phenyl}-propansäure

7. 3-{2-(3-Carboxyphenylmethyloxy)-6-[3-(4-(propen-2-yl)-3-methoxy-2-propylphenoxy)-propoxy]-phenyl}-propansäure

## Claims

1. The invention relates to leukotriene-B₄ antagonists of formula I 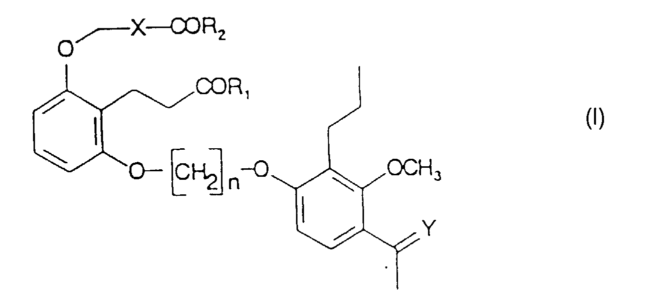 wherein
n is an integer from 2 to 5,
X is a direct bond, from 1 to 6 methylene units, an *ortho-,* meta- or para-substituted phenyl ring or a meta- or para-substituted pyridine ring,
Y is a bond to a hydrogen atom and at the same time to a hydroxy group, or is a doubly-bonded oxygen atom or -O-CH₂-CH₂-O-,
each of R₁ and R₂ is the radical OH, -O-(C₁-C₄)alkyl, -O-(C₃-C₆)cycloalkyl, -O-(C₆-C₁₀)aryl, -O-(C₇-C₁₂)aralkyl, -O-(CH₂)-CO-(C₆-C₁₀)aryl or the radical NHR₃ wherein R₃ is hydrogen, (C₁-C₄)alkyl, (C₃-C₆)cycloalkyl or (C₇-C₁₂)-aralkyl,
and salts thereof with physiologically tolerable bases and cyclodextrin clathrates thereof.

2. Pharmaceutical preparations characterized by a content of leukotriene-B₄ antagonists of the general formula I according to patent claim 1.

3. Process for the preparation of leukotriene-B₄ antagonists of formula I, characterised in that a compound of formula II 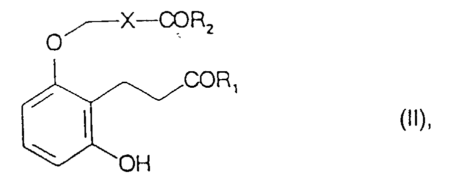 wherein X, R₁ and R₂ are as defined above, is reacted in a manner known *per se* with a compound of formula III 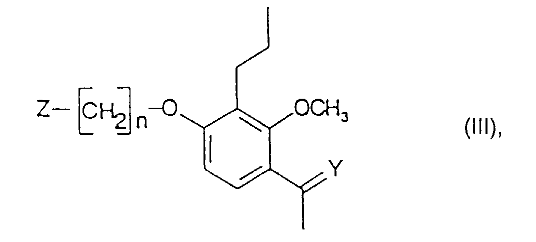 wherein n and Y are as defined above and Z may be either a halogen atom or a sulphonate, such as, for example, tosylate, mesylate or trifluorosulphonate, in the presence of caesium, lithium or potassium carbonate, and, optionally, protecting groups are split off, the methyl ketone is reduced, ester groups are hydrolysed, carboxy groups are esterified or resulting acids of formula I are reacted with organic or inorganic bases or with cyclodextrins.

4. (E/Z)-3-{2-(3-Methoxycarbonylphenylmethyloxy)-6-[3-(4-(1 -methoxypropen-2-yl)-3-methoxy-2-propylphenoxy)-propoxy]-phenyl}-propanoic acid methyl ester.

5. 3-{2-(3-Methoxycarbonylphenylmethyloxy)-6-[3-(4-propen-2-yl)-3-methoxy-2-propylphenoxy)-propoxy]-phenyl}-propanoic acid methyl ester.

6. (E/Z)-3-{2-(3-Carboxyphenylmethyloxy)-6-[3-(4-(1-methoxypropen-2-yl)-3-methoxy-2-propylphenoxy)-propoxy]-phenyl}-propanoic acid.

7. 3-{2-(3-Carboxyphenylmethyloxy)-6-[3-(4-propen-2-yl)-3-methoxy-2-propyl-phenoxy)-propoxy]-phenyl}-propanoic acid.

## Revendications

1. L'invention concerne des antagonistes de la leucotriène B₄ de formule I 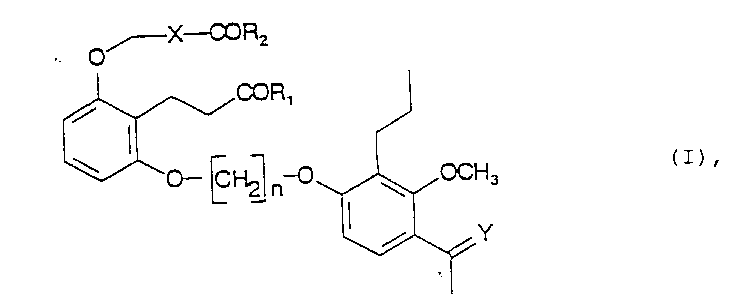 dans laquelle
n est un nombre entier de 2 à 5,
X est une liaison directe, 1 *à 6* motifs méthylène, un cycle phényle substitué en position ortho, méta ou para, ou un cycle pyridine substitué en position méta ou para,
Y est une liaison à un atome d'hydrogène et en même temps à un groupe hydroxy, un atome d'oxygène doublement lié ou -O-CH₂-CH₂-O-,
R₁ et R₂ représentent le reste OH, -O-(alkyle en C₁-C₄), -O-(cycloalkyle en C₃-C₆), -O- (aryle en C₆-C₁₀) , -O-(aralkyle en C₇-C₁₂), -O-(CH₂)-CO-(aryle en C₆-C₁₀) ou le reste NHR₃ avec R₃ étant l'hydrogène, alkyle en C₁-C₄, cycloalkyle en C₃-C₆ ou aralkyle en C₇-C₁₂, ainsi que leurs sels avec des bases physiologiquement tolérées et leurs clathrates de cyclodextrine.

2. Préparations pharmaceutiques caractérisées en ce qu'elles contiennentt des antagonistes de la leucotriène B₄ de formule générale I selon la revendication 1.

3. Procédé pour la préparation des antagonistes de la leucotriène B₄ de formule I caractérisé en ce que l'on fait réagir de façon connue en soi un composé de formule II 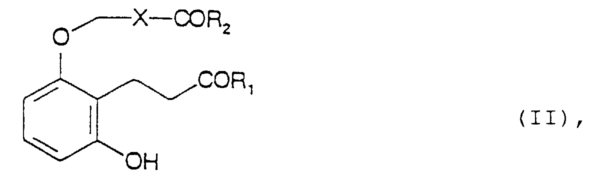 dans laquelle X, R₁ et R₂ ont les significations données ci-dessus avec un composé de formule III 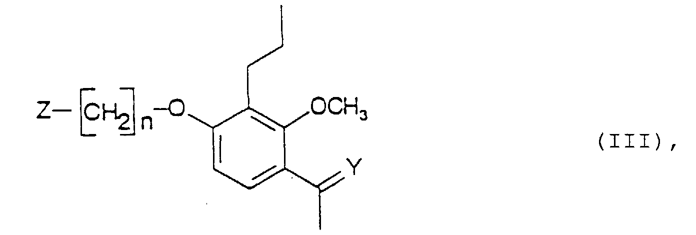 dans laquelle n et Y ont les significations données ci-dessus, et Z représente soit un atome d'halogène soit un sulfonate, comme par exemple le tosylate, le mésylate ou le trifluorosulfonate, en présence de carbonate de césium, de lithium ou de potassium, et on dissocie éventuellement les groupes protecteurs, on réduit la méthylcétone, on saponifie les groupes ester, on estérifie les groupes carboxyle ou on fait réagir les acides de formule I obtenus avec des bases organiques ou inorganiques ou des cyclodextrines.

4. (E/Z)-3-{2-(3-Méthoxycarbonylphénylméthyloxy)-6-[3-(4-(1-méthoxypropèn-2-yl)-3-méthoxy-2-propylphénoxy)-propoxy]-phényl}-propanoate de méthyle

5. 3-{2-(3-Méthoxycarbonylphénylméthyloxy)-6-[3-(4-(propèn-2-yl)-3-méthoxy-2-propylphénoxy)-propoxy]-phényl}-propanoate de méthyle

6. Acide (E/Z)-3-{2-(3-carboxyphénylméthyloxy)-6-[3-(4-(1-méthoxypropèn-2-yl)-3-méthoxy-2-propylphénoxy)-propoxy]-phényl}-propanoïque

7. Acide 3-{2-(3-carboxyphénylméthyloxy)-6-[3-(4-(propèn-2-yl)-3-méthoxy-2-propylphénoxy)-propoxy]-phényl}-propanoïque
